Europäisches Patentamt

European Patent Office

Office européen des brevets

(11) Publication number: **0 260 105 B1**

(19)

# (12) EUROPEAN PATENT SPECIFICATION

(45) Date of publication of patent specification: **18.05.94**

(21) Application number: **87307918.0**

(22) Date of filing: **08.09.87**

(51) Int. Cl.5: **C12N 9/14**, C12P 7/64,
C12N 9/16, C12N 9/20,
C12N 9/50

(54) **Preparation of enzymes having altered activity.**

(30) Priority: **09.09.86 US 905363**
**21.08.87 US 86869**

(43) Date of publication of application:
**16.03.88 Bulletin 88/11**

(45) Publication of the grant of the patent:
**18.05.94 Bulletin 94/20**

(84) Designated Contracting States:
**DE ES FR GB NL SE**

(56) References cited:
**EP-A- 0 130 756**
**EP-A- 0 251 446**
**WO-A-87/05050**

**JOURNAL OF MOLECULAR STRUCTURE
(THEOCHEM.), vol. 123, 1985, Amsterdam
(NL); P.NAGY et al., pp. 413-419***

**BIOSIS DATABASE, 1985; J.A.WELLS et al.,
no. 29034732***

(73) Proprietor: **GENENCOR, INC.**
**180 Kimball Way**
**South San Francisco California 94080(US)**

(72) Inventor: **Arbige, Michael Vincent**
**1256 Birch Street**
**Montara California 94037(US)**
Inventor: **Estell, David Aaron**
**250 Diablo Avenue**
**Mountain View California 94043(US)**
Inventor: **Pepsin, Michael Jay**
**1842 Clement Street**
**San Francisco California 94121(US)**
Inventor: **Poulose, Ayrookaran Joseph**
**2540 Carmel Drive**
**San Bruno California 94066(US)**

(74) Representative: **Armitage, Ian Michael et al**
**MEWBURN ELLIS**
**York House**
**23 Kingsway**
**London WC2B 6HP (GB)**

PROCEEDINGS OF THE NATL. ACADEMY OF SCIENCES USA, vol. 84, March 1987, Washington, DC (US); J.A.WELLS et al., pp. 1219-1223*

CHEMICAL ABSTRACTS, vol. 104, no. 19, 12 May 1986, Columbus, OH (US); D.A.ESTELL et al., p. 130, no. 162674r*

PROCEEDINGS OF THE NATL. ACADEMY OF SCIENCES USA, vol. 84, August 1987, Washington, DC (US); J.A.WELLS et al., pp. 5167-5171*

PROTEIN ENGINEERING, vol. 1, no. 4, August/September 1987, Eynsham, Oxford (GB); B.C.CUNNIGHAM et al., pp. 319-325*

## Description

The present invention relates to a method for modifying enzymes with catalytic triads such that the transesterification rate/hydrolysis rate ratio is altered when the enzymes are used as a catalyst. The present invention also relates to a method of altering nucleophile specificity of enzymes with catalytic triads. The invention further relates to enzymes made by the methods of the invention.

A wide variety of enzymes with catalytic triads are well known. These include serine hydrolases and cystine hydrolases. Broadly, these enzymes include esterases such as proteases, lipases, phospholipases, etc. In general, these enzymes all act to some degree on an ester, forming an alcohol and an acid (hydrolysis) or replacing the ester with a different ester (transesterification, both inter and intra). These two types of reactions are competing reactions and any given enzyme with a catalytic triad will produce both transesterification and hydrolysis if the reaction environment has another nucleophile present besides water. The ratio of the rates for these two reactions is the transesterification/hydrolysis ratio. Specific enzymes may have preference for a specific nucleophile in catalyzed reactions called nucleophile specificity.

Transesterification is useful for a variety of reactions. For example, modifying relatively inexpensive fats and oils to more expensive or desired fats and oils, such as conversion of palm or olive oil to cocoa butter or making fatty acid acid esters and fatty alcohols (cosmetics, surfactants, thickeners, pharmaceuticals).

Likewise, hydrolysis is useful in making of hydrolyzates such as mono or diglycerides, production of free fatty acids, or production of glycerol.

The alteration of nucleophile specificity is important in order to improve the use of enzymes in synthetic reactions in general. When the reactive nucleophile is the amino group of an amino acid, for example, peptides may be easily sythesized.

In a paper by Randall Wood of Texas A&M University, incorporated herein by reference, use of enzymes, particularly lipases and phospholipases, is described for the transesterification and hydrolysis of triglycerides and related lipids. Both inter- and intra-transesterification reactions are described as well as the production of glycerol and mono- and diglycerides by treatment with lipases and phospholipases. Enzymes are selected based on the particular substrate to be attacked as well as the amount of transesterification or hydrolysis which occurs under a given condition.

In Biocatalysis in Organic Synthesis, Macrae (1985) 195-208, a process is described using microbial lipases as catalysts for transesterification of fats and oils by altering the distribution of fatty-acyl groups among the triglycerols. Hydrolysis is described as a side reaction, controlled in part by the amount of water available to the reaction system.

In U. S. Patent 4,416,991, incorporated herein by reference, a method is described for the enzymatic transesterification of a lipid, which comprises continuously or repeatedly contacting an enzyme or an enzyme having transesterification activities with a fresh supply of a dried fatty ester substrate such as fats and oils of glycerides. The method is designed to limit hydrolysis reactions where a desired enzyme is being used.

The hydrolysis of a series of esters of benzoyl-L-arginine and glycylglycine by the hydrolase enzymes chymotrypsin, trypsin, subtilisin, papain and ficen is described in Jour. Bio. Chem. Vol 241, No. 16, pp. 3811-3817 (1966). The article described the inhibition of acid release by the presence of straight chain alcohols. The study suggests that the ability to catalyze alcoholytic reactions is a property of the hydrolase enzymes. The transesterification of amino acids by subtilisins similary was shown by Glazer in Jour. Bio. Chem. Vol. 241, No. 3 (1966). It was shown that transesterification reactions between tyrosine ethyl ester and various alcohols occurred.

Enzymes with catalytic triads are available from a wide variety of sources such as from microbial, plant, insect, or animal. These have a variety of useful properties as well as a variety of transesterification/hydrolysis ratios and nucleophile specificities.

In one aspect this invention provides a method of altering enzymes with catalytic triads so as to change their transesterification/hydrolysis ratio without substantially altering the rest of the reaction conditions. In another aspect the invention provides such enzymes made by the method hereof.

Accordingly, the invention provides a method of altering the transesterification/hydrolysis ratio of an enzyme with a catalytic triad comprising:

a) selecting an enzyme with a catalytic triad;

b) identifying the catalytic triad;

c) selecting an amino acid residue within about 15 angstroms of the identified catalytic triad;

d) replacing the selected amino acid with an amino acid different than originally occurring in the selected enzyme;

and further optionally

3

e) screening the enzyme having a replaced amino acid for transesterification/hydrolysis ratio; and

f) repeating c, d, and e until the optimal desired transesterification/hydrolysis ratio is obtained.

The invention also relates to enzymes made by this method and the transesterification and hydrolysis reactions catalyzed by enzymes made by the method of the invention. It has further been discovered that the method of the invention altering the transesterification/hydrolysis ratio alters the nucleophile specificity of the selected enzyme with a catalytic triad. The invention also relates to a method for altering nucleophile specificity in selected enzymes with catalytic triads.

A) Selection of an enzyme with a catalytic triad.

Enzymes of the invention are those enzymes having catalytic triads such as serine and cystine hydrolases which catalyze reactions at selected sites in the presence of a nucleophile. They generally catalyze the hydrolysis of ester and amide bonds. The substrate (e.g. carboxylic acid ester) reacts with a catalytic amino acid of the catalytic triad such as serine or cystine of a serine or cystine of a serine or cystine hydrolase to give an intermediate in which the active site oxygen forms an ester linkage (acyl enzyme) with the acid group of the substrate. During hydrolysis, water, acting as a nucleophile, attacks the acyl enzyme to give free acid and enzyme. Other nucleophiles such as alcohols, amines, or thiols can also attack the acyl enzyme yielding esters, amides, or thioesters, or the like as products instead of free acids. In general, serine and cystine hydrolases catalyze attack by water in preference to other nucleophiles. Selection of enzymes for this use is so made depending on the specificity of the enzyme by the particular ester or nucleophile as well as general considerations of availability, cost, safety, etc., known to one skilled in the art. The preferred enzymes with catalytic triads are the serine and cystine hydrolases. Serine and cystine hydrolases can be selected from groups such as esterases comprising proteases, lipases, phospholipases, etc. See also, Enzymatic Nomenclature (1979) p. 234-274 and 352-355 for a futher list of esterases. Examples of suitable proteases include papain, cathepsin, bromelain, chymotrypsin, and subtilisin. Preferred proteases are the subtilisins, especially Subtilisin BPN′. Examples of lipases include animal lipases such as pancreatic, lipoprotein, hepatic, lingual, etc; microbial lipases such as lipases from Pseudomonas fragi, Pseudomonas fluorescens, Staphylococcus aureus, Candida lipolytica, Geotrichum candidum, Penicillium roqueforti, Aspergillus flavus, Aspergillus niger, Aspergillus oryzae, Rhizopus oligosporus, Rhizopus delemar, Rhizopus arrhizus, Mucor javanicus, Torulopsis ernobii, Penicillium cyclopium, Candida cylindracea, Candida paralipolytica, Leptospira pomona, Leptospira biflexa, Corynebacterium acne. Preferred esterases are those from Bacillus species such as B.subtilis, and include enzymes such as subtilisin BPN', subtilisin Carlsberg, and the like. Lipases for use in the invention also include lipases from other sources such as Ricinas communis, Veronona anthelmintica, Locusta migatoria, Peri-planteta americana, Phiosamia ricini, and Diatraea grandiosella. Other enzymes with catalytic triads can readily be identified by one skilled in the art. It is anticipated that as new enzymes are discovered with catalytic triads that these too will be within the teaching of the present invention.

B) Identification of the Catalytic Triad.

The active site of an enzyme such as a serine and cystine hydrolase is made up of three amino acids--aspartic acid, histidine, and (serine or cystine), in serine and cystine hydrolases -- called the catalytic triad. One of the catalytic amino acids of the catalytic triad reacts with the substrate carbonyl to give the acyl enzyme which can then break down either through attack by water (hydrolysis) or attack by a different nucleophile such as an alcohol (alcoholysis-transesterification). See Enzymatic Reaction Mechanisms, by C. Walsh, Freeman & Co., 1979 for a discussion of catalytic triad. The catalytic triad can be identified from the crystal structure or from chemical methods (see Means, G., and Feeny, R., Chemical Modification of Proteins, Holden-Day, Inc.).

C) Selection of the codon for replacement.

It has been surprisingly discovered that replacement of an amino acid within about 15 angstroms of a catalytic triad of an enzyme having a catalytic triad will lead to an increase or decrease in nucleophile preference and hence change the transesterification/hydrolysis ratio. Replacement of an amino acid greater than about 15 angstroms from the catalytic triad has little or no effect on the transesterification/hydrolysis ratio or nucleophile preference.

Enzymes have numerous folds and bends in the structure and one would in general use the crystal structure of the enzyme to determine which amino acids are within 13 angstroms of the catalytic triad regardless of the primary structure of the enzyme. Where no crystal structure is available, positions in the primary sequence about 6 amino acids on either side of an amino acid of the catalytic triad would be within the 15 angstrom requirement.

D) Replacement of the selected amino acid.

Once a site is selected within the considerations of the invention, the amino acid may be replaced by any other amino acid usually other than the naturally occurring amino acid. While it is not possible to predict if the substitution will lead to an increase or decrease in the ratio, applicants have discovered that either an increase or decrease will occur. Preferred substitution include the substitutions in subtilisin and equivalent sites around the catalytic serine 221 including positions 222 and 217 in subtilisin. See U.S. Patent Serial No. 614,612 (EP 130756) and substitutions in lipase at positions around two of the amino acids of the catalytic triad at ser 126 or cys 126 and His 206.

E) Screening.

To determine to what degree and which way the transesterification/hydrolysis ratio or nucleophile specificity is modified, the enzyme is screened and compared to values prior to the mutation. The exact method will depend on the exact enzyme reaction being conducted and which nucleophiles are desired but generally consists of measuring the amount of transesterification product and hydrolysis product under a set of fixed conditions both prior and after the mutation of the enzyme using a selected nucleophile. One skilled in the art would be able to suitably select a method for so doing or could use the procedure used in the examples noted (substituting appropriate reagents, conditions, enzymes, etc.). The screen could consist of any ester as stock substrate in a reaction mix containing any nucleophile or nucleophiles of interest that the operator(s) would like to see substituted at the site of attack.

F) Repetition of the mutations

In order to obtain a mutant enzyme with the best ratio or nucleophile specificity possible in the desired direction, more than one amino acid substitution at a given selected site in the enzyme can be made and the values compared. All nineteen amino acid substitutions could be made to determine which ratio will best suit the users needs. Further, substitutions at more than one site within the parameters of the invention can be done in order to further optimize the results. As stated before, however, no mutations outside the 15 angstrom range will produce a significant effect on a transesterification/hydrolysis ratio or nucleophile specificity. Further, care must be made to preserve the catalytic triad function. For example, substitution by non-hydroxyl or non-sulfhydroxyl containing amino acids at the catalytic amino acid will inactivate the enzyme entirely.

The enzymes made thus are useful catalysts in transesterification reactions, hydrolysis reactions, and for specific nucleophile attack. Where one desires primarily one type of reaction an enzyme may be selected which favors the desired reaction and/or nucleophile when compared with the starting enzyme. It should be observed however, that since only a limited number of substitutions may be made at the selected locations, and as such it is possible, however unlikely, that the starting enzyme will have the desired or maximum result. The method does assure that the ratio or nucleophile specificity may be changed in at least one direction.

The following examples are representative of the invention and not intended to be limiting. One skilled in the art would be able to make changes in the methods, procedures, enzymes selected, etc. based on the disclosure herein.

Materials and Methods

Gas Chromatography

All gas chromatography work was done on a Hewlett-Packard 5880A series gas chromatograph fitted with flame ionization detector and a Hewlett-Packard 7672A automatic sampler. For detection of both amino acid esters and ethyl acetate a six foot prepacked Supelco 2mm ID glass column packed with 10% OV-11 on 100/120 Supelcoport was used according to the method of Gehrke and Leimer (1). Gas flow rate conditions for amino acid esters were as follows: hydrogen, 38 ml min$^{-1}$ and helium 30 ml min.$^{-1}$. For ethyl acetate detection the flow rate of helium was 20 ml min.$^{-1}$.

5

For the detection of ethyl-acetate injection and detector ports were heated heated to 150°C. A temperature rate program starting at 50°C increasing by 2C min⁻¹ up to 80°C was used for separation of these compounds. For the detection of amino acid esters, injection and detection ports were heated to 250°C. A temperature program, starting at 230°C increasing by 3°min⁻¹ up to 280°C was used for separation.

## Sample Preparation

All standards, ethyl-acetate (Aldrich Chemicals), amino acid esters (Sigma Chemical) were prepared fresh before each set of experiments and quantified on the GC. Normal volume of sample injection was 3ul.

The wild type and mutant subtilisin enzymes Met-->Phe-222 (F-222), Met -->Glu-222 (Q-222). Tyr--->Leu-217 (L-217) were prepared as described in Serial No. 614,612 filed May 29, 1984, (EP 130756). The catalytic serine is at position +221.

## Amino Acid Esters

Amino acid esters were prepared at a concentration of .05M in milliQ water at a pH of 6.3. A 10% methanol solution was prepared in 0.2M MES buffer at a pH of 6.3. 2.5ml of each reagent was added to 10ml glass screw cap test tubes and the temperature was allowed to equilibrate at 36°C in a water bath. Final concentration of amino acid esters and alcohol was 0.025M and 5% respectively.

Enzyme was added to this solution at a final concentration of .04mg ml⁻¹ or .08mg ml⁻¹. Samples (75ul) were taken from the reaction mixture over fixed time intervals and an equal volume of 0.1N HCl was added. The samples were frozen until analysis by G.C. Transesterification hydrolysis ratios were determined from this amount of tyrosine methylester and free tyrosine formed from tyrosine ethylester. Controls consisted of sample without enzyme and without alcohol, but all other components were included. Experimental values were corrected for any nonenzymatic hydrolysis or transesterification.

## Triglycerides

Triacetin and ethanol were used for model transesterification demonstrations of triglycerides. All reactions were carried out at constant pH by a pH stat. Equal volumes of triacetin (2.25%) and anhydrous ethanol (6.0%) were added to a jacketed titration beaker (2ml volume) held at 36°C by water circulation through a Brinkman RM6 water bath. The reaction vessel was part of a potentiometric automatic titration (pH stat), model AT-118 (Kyoto Electronics). The initial pH of the reaction was adjusted to 6.4 (if needed) and a zero time sample enzyme removed. Enzyme was added to the mixture at a final concentration of 0.02 or 5.0 mg ml⁻¹ via a hamilton glass syringe. When the reaction pH dropped to 6.29 the titrator was started and set to hold the pH at 6.3 by the addition of 0.IN NaOH. Sampling consisted of removing 75ul of the reaction mixture with a Hamilton syringe and adding an equal volume of 0.24N HCl. Samples were frozen until analysis. Hydrolysis data was automatically calculated by the titrator as mg/ml min⁻¹ of acetic acid released.

## RESULTS

## Amino acid hydrolysis/Transesterification

## Wild Type Enzyme

Table 1A&B depicts the results of hydrolysis and transesterification (tyr-methyl-ester formation) when several levels of the wild type enzyme (subtilisin BPN′) were incubated in a .025M tyr-ethylester solution containing 5% methanol at 36°C over time. For this substrate the WT enzyme has a transesterification to hydrolysis ratio of approximately 0.6. This ratio is apparent both at .04mg ml⁻¹ of enzyme and at .08 mg ml⁻¹, indicating that the hydrolysis/transesterification ratio is essentially independent of enzyme concentration (the hydrolysis rate with the larger enzyme conc. increased by over 60%).

## Mutant Enzymes

Studies of mutant subtilisins showed that amino acid replacements could lead to alterations in the topology of the active site. In order to determine if these effects could alter nucleophile specificity (for e.g.

to improve the rate of attack by an alcohol vs. water) we chose the following mutant enzymes for study: F-222, Q-222, and L-217. Crystal structures of these enzymes showed definite alterations of their active sites relative to the wild type enzyme which made them ideal for this study.

Fig. 2A shows the transesterificaton/hydrolysis rates for the F-222 mutant enzyme under the same set of conditions reported above for the wild type enzyme. F-222 enzyme at 0.4mg/ml has a transesterification/hydrolysis ratio of 1.6 (2.6 fold greater than the wild type enzyme) while its hydrolysis rate was roughly half that of the wild type enzyme (0.094).

For the Q-222 mutant enzyme (Table 2B) the hydrolysis rate was much lower than the wild type enzyme (over 10 fold lower). Because of the slow hydrolysis rate it took approximately 240 minutes to hydrolyze over 40% of the substrate. The transesterification/hydrolysis ratio over this time period was only 0.14 (4.3 fold lower than the wild type enzyme).

These data show that amino acid replacements in the vicinity of position 221 (the active site serine) can radically change the nucleophile specificity from that of the wild type enzyme.

## Triglyceride Studies

Triacetin was chosen as a model triglyceride to study because it is relatively soluble and subtilisin has been shown to have reasonable hydrolytic activity against this substrate. For these studies, a third mutant L-217 was chosen in addition to F-222 and Q-222, because it represents a site on the other side of the active site within the 15A° radius of the catalytic serine.

Fig. 3A and 3B show that for this substrate a similar change in nucleophile specificity is evident with the mutant enzymes with respect to the wild type enzyme. The data in this table is reported as ratios of ethyl acetate formation to free acetic acid. In this series of experiments, L-217 showed the greatest hydrolysis rate followed by the wild type enzyme, F-222, and then Q-222. In this case, it is also apparent that the hydrolysis rates have no correlation with ethanolysis rates. For this substrate both L-217 and Q-222 mutant enzymes greatly prefer hydrolysis over ethanolysis. F-222 again shows a distinct preference for the alcohol as the attacking nucleophile over water.

## References

1. Gehrke and Leimer. J. Chromatog. 58:219 (1971).
2. Macrae, A.R. Biocatalysts in Organic Syntheses. Proceedings of an International Symposium held at Noordwijkerhout, The Netherlands, 14-17. April 1985. Elsevier Science Publishers.

## Amino Acid Transesterification/Hydrolysis
### Wild Type Enzyme

tyr-ethyl ester In 5% MeOH

Wild Type Enzyme 0.04 mg ml$^{-1}$

tyr-ethyl ester In 5% MeOH

Wild Type Enzyme .08 mg ml$^{-1}$

| Time mins. | % hyd | mg ml$^{-1}$ methylester | | Rates Hyd. | mg/ml min$^{-1}$ ME Formation | TE/Hyd | Time mins. | % hyd | mg ml$^{-1}$ methylester | | Rates Hyd | mg/ml min$^{-1}$ ME formation | TE/Hyd |
|---|---|---|---|---|---|---|---|---|---|---|---|---|---|
| 1 | 6.3 | 0.12 | | | | | 1 | 8.9 | 0.28 | | | | |
| 5 | 17.8 | 0.40 | | | | | 5 | 25.0 | 0.71 | | | | |
| 10 | 32.1 | 0.79 | | | | | 10 | 45.0 | 1.11 | | | | |
| 15 | 46.5 | 0.97 | | | | | 15 | 65.0 | 1.08 | | | | |
| 20 | 61.0 | 1.25 | | | | | 20 | 85.5 | 1.09 | | | | |
| | | | | 0.094 | 0.056 | .60 | | | | | 0.156 | 0.090 | .58 |

Table 1A

Table 1B

EP 0 260 105 B1

## Amino Acid Transesterification/Hydrolysis
### Mutant Enzymes

F-222  0.04 mg ml$^{-1}$

| Time mins. | % hyd | mg ml$^{-1}$ methylester | Rates Hyd. | mg/ml min$^{-1}$ ME Formation | TE hyd |
|---|---|---|---|---|---|
| 1 | 19.4 | 0.25 | | | |
| 5 | 24.0 | 0.52 | | | |
| 10 | 30.0 | 0.86 | | | |
| 15 | 35.6 | 1.20 | | | |
| 20 | 41.4 | 1.54 | | | |
| | | | .043 | .068 | 1.6 |

Table 2A

Q-222  .04 mg ml$^{-1}$

| Time mins. | % hyd | mg ml$^{-1}$ methylester | Rates Hyd | mg/ml min$^{-1}$ ME formation | TE hyd |
|---|---|---|---|---|---|
| 1 | 1.6 | 0.00 | | | |
| 5 | 2.4 | | | | |
| 10 | 3.2 | | | | |
| 15 | 4.1 | | | | |
| 20 | 5.0 | | | | |
| 240 | 44.0 | 0.27 | | | |
| | | | .008 | .001 | 0.13 |

Table 2B

## Ethyl Acetate Formation
### Irlacetin in 6% Ethanol

**Transesterification/Hydrolysis Ratio Product**

5 mg/ml of enzyme

| Time (mins) | Q-222 | E-222 | WT |
|---|---|---|---|
| 10 | -- | 1.78 | 1.48 |
| 20 | -- | 1.36 | 1.03 |
| 30 | .011 | 1.16 | 0.86 |
| 45 | .027 | 1.00 | 0.73 |
| 60 | .038 | 0.91 | 0.66 |
| 90 | .047 | 0.86 | 0.59 |
| Hydrolysis rates mg/ml min$^{-1}$ of acetic acid formed | .0013 | 0.0064 | 0.0098 |

Table 3A

**Transesterification/Hydrolysis Ratio Product**

.02 mg/ml of enzyme

| Time (mins) | L-217 | E-222 | WT |
|---|---|---|---|
| 30 | -- | 3.21 | 1.21 |
| 60 | -- | 2.73 | 1.28 |
| 90 | -- | 2.48 | 1.35 |
| 120 | .16 | 2.32 | 1.38 |
|  | .0008 | 0.0002 | 0.0003 |

Table 3B

* In both 5 mg ml$^{-1}$ exps and .02 mg ml$^{-1}$ exps the hydrolysis capacity of the wild type enzyme was exactly 1.5 times that of the F-222 enzyme preps.

Cystine Hydrolases (Subtilisin)

The active site serine 221 of subtilisin BPN' and its variant enzyme, F222, was chemically modified to a cysteine residue using the methods of Polgar & Bender (1967) Biochemistry 6:610-620. Thiolsubtilisin reportedly does not stabilize the transition state as well as subtilisin during acylation of specific amide or ester substrates (Phillipp & Bender (1983) Molecular and Cellular Biochemistry 51:5-32). Hence, the relative rates of transesterification and the transesterification to hydrolysis ratios were measured for the cysteine 221 containing native and F222 enzymes using the substrates tyrosine methyl ester and tyrosine ethyl ester in the presence of 10% ethanol or methanol respectively. The data given below shows that the chemically modified F222 has higher ratios of transesterification to hydrolysis than does the modified native enzyme for formation of both tyrosine esters. In addition, of the two modified enzymes, F222 has the higher relative rates of transesterification. Of interest is the finding that the difference in the ability to transesterify is greater for the modified enzymes than it is for the unmodified enzymes. Although lower in activity compared to serine hydrolases, the thiolsubtilisin form of these enzymes is still capable of catalyzing transesterification reactions and the F222 variant remains the better catalyst.

Tyrosine transesterification using the chemically derived thiolsubtilisin form of wild type enzyme and its F222 variant:

| tyrosine ester in 10% alcohol + 0.3mg/ml enzyme | | | | |
|---|---|---|---|---|
| | tyrosine ethyl ester formation | | tyrosine methyl ester formation | |
| | TE/Hyd. | rel. TE rate | Te/Hyd. | rel. TE rate |
| F222/C221 | 3 | 11 | 8 | 22 |
| WT/C221 | 2 | 1 | 1.2 | 1 |

Tyrosine transesterification using wild type subtilisin and its F222 variant:

| tyrosine ester in 10% alcohol + 0.03mg/ml enzyme | | | | |
|---|---|---|---|---|
| | tyrosine ethyl ester formation | | tyrosine methyl ester formation | |
| | Te/HYd. | rel. TE rate | TE/HYd. | rel. TE rate |
| F222 | 3.3 | 1.6 | 13 | 1.6 |
| WT | 1.5 | 1 | 5 | 1 |

Serine and Cystine Hydrolase of Lipase

The active sites in lipase are Ser 126 or Cys 126 and 206 his. Modifications of the type used for subtilisin was done for lipase to show the applicability of the invention to other enzymes with catalytic triads. Following are the results of these experiments.

Various times (1/2 hr. - 2 hr.) in solution of a 1% emulsion of trioctanoin/polyvinyl alcohol (1:1) and 10% methanol.

| Wild Type (WT) | C8-Me | C8-Ac | Ratio |
|---|---|---|---|
| lipase | 1.19 | 2.87 | 0.41 |
| val 127 | 1.33 | 2.55 | 0.52 |
| ser 127 | 1.45 | 2.6 | 0.56 |
| leu 127 | 1.45 | 2.44 | 0.59 |
| cys 127 | 0.85 | 2.55 | 0.33 |
| asn 127 | 1.02 | 1.79 | 0.57 |
| arg 127 | 0.81 | 2.55 | 0.32 |
| gly 127 | 0.98 | 2.58 | 0.38 |
| thr 127 | 1.6 | 2.54 | 0.63 |
| cys 126 | 0.95 | 1.64 | 0.58 |
| ala 207 | 1.38 | 2.4 | 0.58 |

Various times in solution of a 1% emulsion of trioctanoin/polyvinyl alcohol (1:1) and 10% glycerol.

| | C8-Me | C8-Ac | Ratio |
|---|---|---|---|
| wt | 0.19 | 1.22 | 0.16 |
| ala 207 | 0.28 | 1.31 | 0.21 |
| cys 126 | 0.14 | 0.3 | 0.47 |
| thr 127 | 0.26 | 0.97 | 0.27 |

At times listed in 95% emulsion of trioctenoin/SDS (10:1) and 10% methanol.

| | 1/4 Hour | | | 1/2 Hour | | |
|---|---|---|---|---|---|---|
| | C8-Me | C8-acid | Ratio | C8-Me | C8-acid | Ratio |
| wt | 5224 | 4030 | 1.30 | 8374 | 7222 | 1.16 |
| ser 127 | 11582 | 6121 | 1.89 | 15516 | 12160 | 1.28 |
| thr 127 | 14784 | 7284 | 2.04 | 20357 | 12629 | 1.61 |
| ser 127/ala 207 | 2107 | 1093 | 1.93 | 3356 | 2432 | 1.40 |
| thr 127/ala 207 | 3861 | 3042 | 1.27 | 7300 | 4216 | 1.73 |
| arg 127 | 2613 | 2616 | 0.98 | 4111 | 3870 | 1.06 |
| gln 205 | 41bg | 3598 | 1.16 | 6531 | 5359 | 1.22 |
| thr 207 | 423 | ------- | | 654 | 430 | 1.52 |
| asn 205 | 6011 | 4992 | 1.20 | 10850 | 7982 | 1.36 |
| val 207 | 1234 | 822 | 1.50 | 2041 | 1352 | 1.51 |

12

**Claims**

**Claims for the following Contracting States : DE, FR, GB, NL, SE**

1. A method of altering the transesterification/hydrolysis ratio or nucleophile specificity of an enzyme having a catalytic triad comprising:
   a) selecting an enzyme with a catalytic triad;
   b) identifying the catalytic triad in the enzyme;
   c) selecting an amino acid residue within 15 angstroms of the identified catalytic triad; and
   d) replacing the selected amino acid with an amino acid different than originally occuring in the selected enzyme.

2. A method according to Claim 1 which further comprises the step of e) screening the enzyme having a replaced amino acid for transesterification/hydrolysis ratio or nucleophile specificity.

3. A method according to Claim 2 which further comprises the step of f) repeating c, d, and e until the optimal desired transesterification/hydrolysis ratio or nucleophile specificity is obtained.

4. A method according to any one of claims 1, 2 and 3 wherein the enzyme is a serine or cystine hydrolase.

5. A method according to any one of claims 1, 2 and 3 wherein the enzyme is an esterase selected from protease, a lipase or phospholipase.

6. A method according to Claim 5 wherein the esterase selected is a lipase.

7. A method according to Claim 6 wherein the esterase is a subtilisin.

8. A method according to Claim 7 wherein the subtilisin is subtilisin BPN'.

9. A method of altering the transesterification/hydrolysis ratio of a first lipase enzyme having a catalytic triad such method comprising:
   (a) replacing an amino acid in the first lipase enzyme which is from 1-6 amino acids away from a catalytic amino acid of the catalytic triad, with a different amino acid to produce a second lipase enzyme;
   (b) determining the transesterification/hydrolysis ratio of said second lipase enzyme; and
   (c) selecting said second lipase enzyme wherein said ratio is altered when compared to the first lipase enzyme.

10. A method of altering the nucleophile specificity of a first lipase enzyme having a catalytic triad, such method comprising:
    (a) replacing an amino acid in the first lipase enzyme which is from 1-6 amino acids away from a catalytic amino acid of the catalytic triad with a different amino acid to produce a second lipase enzyme;
    (b) determining the nucleophile specificity of said second lipase enzyme; and
    (c) selecting said second lipase enzyme wherein said nucleophile specificity is altered when compared to the first lipase enzyme.

11. A method according to claim 9 wherein the amino acid replaced in said first lipase enzyme is one amino acid away from a catalytic amino acid of the catalytic triad of said first lipase enzyme.

12. A method according to claim 10 wherein the amino acid replaced in said first lipase enzyme is one amino acid away from a catalytic amino acid of the catalytic triad of said first lipase enzyme.

13. An enzymatically active mutant lipase enzyme derived from a precursor lipase enzyme having a catalytic triad, said mutant lipase comprising a replacement of an amino acid from 1-6 amino acids away from a catalytic triad amino acid to an amino acid different than the precursor lipase enzyme wherein said mutant lipase exhibits a change in the transesterification/hydrolysis ratio when compared to said precursor lipase enzyme.

**14.** An enzymatically active mutant lipase enzyme derived from a precursor lipase enzyme having a catalytic triad, said mutant lipase enzyme comprising a replacement of an amino acid from 1-6 amino acids away from a catalytic triad amino acid to an amino acid different than the precursor lipase enzyme wherein said mutant lipase enzyme exhibits a change in the nucleophile specificity when compared to the precursor lipase enzyme.

**15.** A mutant enzyme according to claim 13 wherein the amino acid replaced in said precursor lipase enzyme is one amino acid away from a catalytic amino acid of the catalytic triad of said first lipase enzyme.

**16.** A mutant enzyme according to claim 14 wherein the amino acid replaced in said precursor lipase enzyme is one amino acid away from a catalytic amino acid of the catalytic triad of said precursor lipase enzyme.

**17.** A method which comprises, following the identification of an altered enzyme according to any one of claims 1 to 12, and optionally the manufacture of such an enzyme, catalyzing a reaction with said enzyme.

**18.** The use of a mutant enzyme of any one of claims 13 to 16 in a catalytic reaction.

**19.** Following the identification of an altered enzyme according to any one of claims 1 to 12, the manufacture of said enzyme.

**Claims for the following Contracting State : ES**

**1.** A method of altering the transesterification/hydrolysis ratio or nucleophile specificity of an enzyme having a catalytic triad comprising:
a) selecting an enzyme with a catalytic triad;
b) identifying the catalytic triad in the enzyme;
c) selecting an amino acid residue within 15 angstroms of the identified catalytic triad; and
d) replacing the selected amino acid with an amino acid different than originally occuring in the selected enzyme.

**2.** A method according to Claim 1 which further comprises the step of e) screening the enzyme having a replaced amino acid for transesterification/hydrolysis ratio or nucleophile specificity.

**3.** A method according to Claim 2 which further comprises the step of f) repeating c, d, and e until the optimal desired transesterification/hydrolysis ratio or nucleophile specificity is obtained.

**4.** A method according to any one of claims 1, 2 and 3 wherein the enzyme is a serine or cystine hydrolase.

**5.** A method according to any one of claims 1, 2 and 3 wherein the enzyme is an esterase selected from protease, a lipase or phospholipase.

**6.** A method according to Claim 5 wherein the esterase selected is a lipase.

**7.** A method according to Claim 6 wherein the esterase is a subtilisin.

**8.** A method according to Claim 7 wherein the subtilisin is subtilisin BPN'.

**9.** A method of altering the transesterification/hydrolysis ratio of a first lipase enzyme having a catalytic triad such method comprising:
(a) replacing an amino acid in the first lipase enzyme which is from 1-6 amino acids away from a catalytic amino acid of the catalytic triad, with a different amino acid to produce a second lipase enzyme;
(b) determining the transesterification/hydrolysis ratio of said second lipase enzyme; and

14

(c) selecting said second lipase enzyme wherein said ratio is altered when compared to the first lipase enzyme.

10. A method of altering the nucleophile specificity of a first lipase enzyme having a catalytic triad, such method comprising:

(a) replacing an amino acid in the first lipase enzyme which is from 1-6 amino acids away from a catalytic amino acid of the catalytic triad with a different amino acid to produce a second lipase enzyme;

(b) determining the nucleophile specificity of said second lipase enzyme; and

(c) selecting said second lipase enzyme wherein said nucleophile specificity is altered when compared to the first lipase enzyme.

11. A method according to claim 9 wherein the amino acid replaced in said first lipase enzyme is one amino acid away from a catalytic amino acid of the catalytic triad of said first lipase enzyme.

12. A method according to claim 10 wherein the amino acid replaced in laid first lipase enzyme is one amino acid away from a catalytic amino acid of the catalytic triad of said first lipase enzyme.

13. A method which comprises, following the identification of an altered enzyme according to any one of claims 1 to 12, and optionally the manufacture of such an enzyme, catalyzing a reaction with said enzyme.

14. The use of an altered enzyme of any one of claims 1 to 12 in a catalytic reaction.

15. Following the identification of an altered enzyme according to any one of claims 1 to 12, the manufacture of said enzyme.

**Patentansprüche**
**Patentansprüche für folgende Vertragsstaaten : DE, FR, GB, NL, SE**

1. Verfahren zum Verändern des Umesterungs/Hydrolyse-Verhältnisses oder der nukleophilen Spezifität eines Enyzms mit einer katalytischen Triade, umfassend:

a) Auswählen eines Enzyms mit einer katalytischen Triade;

b) Identifizieren der katalytischen Triade im Enzym;

c) Auswählen eines Aminosäurerests innerhalb von 15 Angström von der identifizierten katalytischen Triade; und

d) Ersetzen der ausgewählten Aminosäure durch eine andere Aminosäure, als sie ursprünglich im ausgewählten Enzym vorkommt.

2. Verfahren nach Anspruch 1, das weiters den Schritt des e) Screenens des eine ersetzte Aminosäure aufweisenden Enzyms hinsichtlich des Umesterungs/Hydrolyse-Verhältnisses oder der nukleophilen Spezifität umfaßt.

3. Verfahren nach Anspruch 2, welches weiters den Schritt des f) Wiederholens von c, d und e umfaßt, bis das optimale erwünschte Umesterungs/Hydrolyse-Verhältnis oder die optimale erwünschte nukleophile Spezifität erreicht ist.

4. Verfahren nach einem der Ansprüche 1, 2 und 3, worin das Enzym ein Serin- oder eine Cystinhydrolase ist.

5. Verfahren nach einem der Ansprüche 1, 2 und 3, worin das Enzym eine aus Protease, einer Lipase oder Phospholipase ausgewählte Esterase ist.

6. Verfahren nach Anspruch 5, worin die ausgewählte Esterase eine Lipase ist.

7. Verfahren nach Anspruch 6, worin die Esterase ein Subtilisin ist.

8. Verfahren nach Anspruch 7, worin das Subtilisin Subtilisin BPN' ist.

**9.** Verfahren zum Verändern des Umesterungs/Hydrolyse-Verhältnisses eines ersten Lipaseenzyms mit einer katalytischen Triade, welches Verfahren folgende Schritte umfaßt:

(a) Ersetzen einer Aminosäure im ersten Lipaseenzym, die um 1-6 Aminsäuren von einer katalytischen Aminosäure der katalytischen Triade entfernt ist, durch eine davon verschiedene Aminosäure, um ein zweites Lipaseenzym herzustellen;

(b) Bestimmen des Umesterungs/Hydrolyse-Verhältnisses des genannten zweiten Lipaseenzyms; und

(c) Auswählen des genannten zweiten Lipaseenzyms, bei dem das genannte Verhältnis im Vergleich zum ersten Lipaseenzym verändert ist.

**10.** Verfahren zum Verändern der nukleophilen Spezifität eines ersten Lipaseenzyms mit einer katalytischen Triade, welches Verfahren folgende Schritte umfaßt:

(a) Ersetzen einer Aminosäure im ersten Lipaseenzym, das von 1-6 Aminosäuren von einer katalytischen Aminosäure der katalytischen Triade entfernt ist, durch eine davon verschiedene Aminosäure, um ein zweites Lipaseenzym herzustellen;

(b) Bestimmen der nukleophilen Spezifität des genannten zweiten Lipaseenzyms; und

(c) Auswählen des genannten zweiten Lipaseenzyms, worin die genannte nukleophile Spezifität im Vergleich zum ersten Lipaseenzym geändert ist.

**11.** Verfahren nach Anspruch 9, worin die im genanntemn ersten Lipaseenzym ersetzte Aminosäure um eine Aminosäure von einer katalytischen Aminosäure der katalytischen Triade der genannten ersten Lipaseenzyms entfernt ist.

**12.** Verfahren nach Anspruch 10, worin die in genanntem ersten Lipaseenzym ersetzte Aminosäure eine Aminosäure von einer katalytischen Aminosäure der katalytischen Triade des genannten ersten Lipaseenzyms enfernt ist.

**13.** Enzymatisch aktives, verändertes Lipaseenzym, das von einem Vorläufer-Lipaseenzym mit einer katalytischen Triade abgeleitet ist, wobei die genannte veränderte Lipase eine Ersetzung einer um 1-6 Aminosäuren von einer katalytischen Triaden-Aminosäure entfernten Aminosäure durch einer Aminosäure umfaßt, die unterschiedlich zum Vorläufer-Lipaseenzym ist, wobei die genannte veränderte Lipase eine Veränderung des Umesterungs/Hydrolyse-Verhältnisses im Vergleich zu genanntem Vorläufer-Lipaseenzym aufweist.

**14.** Enzymatisch aktives verändertes Lipaseenzym, das von einem Vorläufer-Lipaseenzym mit einer katalytischen Triade abgeleitet ist, wobei das genannte Lipaseenzym eine Ersetzung einer um 1-6 Aminosäuren von einer katalytischen Triaden-Aminosäure entfernten Aminosäure durch eine Aminosäure umfaßt, die unterschiedlich vom Vorläufer-Lipaseenzym ist, wobei das genannte veränderte Lipaseenzym eine Veränderung in der nukleophilen Spezifität im Vergleich zum Vorläufer-Lipaseenzym aufweist.

**15.** Verändertes Enzym nach Anspruch 13, worin die im genannten Vorläufer-Lipaseenzym ersetzte Aminosäure um eine Aminosäure von einer katalytischen Aminosäure der katalytischen Triade des genannten ersten Lipaseenzyms entfernt ist.

**16.** Geändertes Enzym nach Anspruch 14, worin die in genanntem Vorläufer-Lipaseenzym ersetzte Aminosäure eine Aminosäure von einer katalytischen Aminosäure der katalytischen Triade des genannten Vorläufer-Lipaseenzyms entfernt ist.

**17.** Verfahren, das im Anschluß an die Veränderung eines Enzyms gemäß dem Verfahren nach einem der Ansprüche 1 bis 12 und wahlweise im Anschluß an die Herstellung eines solchen Enzyms das Katalysieren einer Reaktion mit dem genannten Enzym umfaßt.

**18.** Verwendung eines veränderten Enzyms nach einem der Ansprüche 13 bis 16 in einer katalytischen Reaktion.

**19.** Herstellung des genannten Enzyms im Anschluß an die Änderung eines Enzyms gemäß dem Verfahren nach einem der Ansprüche 1 bis 12.

EP 0 260 105 B1

**Patentansprüche für folgenden Vertragsstaat : ES**

1. Verfahren zum Verändern des Umesterungs/Hydrolyse-Verhältnisses oder der nukleophilen Spezifität eines Enyzms mit einer katalytischen Triade, umfassend:
   a) Asuwählen eines Enzyms mit einer katalytischen Triade;
   b) Identifizieren der katalytischen Triade im Enzym;
   c) Auswählen eines Aminosäurerests innerhalb von 15 Angström der identifizierten katalytischen Triade; und
   d) Ersetzen der ausgewählten Aminosäure durch eine andere Aminosäure als sie ursprünglich im augewählten Enzym vorkommt.

2. Verfahren nach Anspruch 1, das weiters den Schritt des e) Screenens der Enzyms mit einer ersetzten Aminosäure hinsichtlich des Umesterungs/Hydrolyse-Verhältnisses oder der nukleophilen Spezifität umfaßt.

3. Verfahren nach Anspruch 2, das weiters den Schritt des f) Wiederholens von c, d und e umfaßt, bis das optimale erwünschte Umesterungs/Hydrolyse-Verhältnis oder die optimale erwünschte nukleophile Spezifität erreicht ist.

4. Verfahren nach einem der Ansprüche 1, 2 und 3, worin das Enzym ein Serin- oder eine Cystinhydrolase ist.

5. Verfahren nach einem der Ansprüche 1, 2 und 3, worin das Enzym eine aus Protease, einer Lipase oder Phospholipase ausgewählte Esterase ist.

6. Verfahren nach Anspruch 5,worin die ausgewählte Esterase eine Lipase ist.

7. Verfahren nach Anspruch 6, worin die Esterase ein Subtilisin ist.

8. Verfahren nach Anspruch 7, worin das Subtilisin Substilisin BPN' ist.

9. Verfahren zum Verändern des Umesterungs/Hydrolyse-Verhältnisses eines ersten Lipaseenzyms mit einer katalytischen Triade, welches Verfahren die folgenden Schritte umfaßt:
   (a) Ersetzen einer Aminosäure im ersten Lipaseenzym, das von 1-6 Aminosäuren von einer katalytischen Aminosäure der katalytischen Triade entfernt ist, durch eine davon verschiedene Aminosäure, um ein zweites Lipaseenzym herzustellen;
   (b) Bestimmen des Umesterungs/Hydrolyse-Verhältnisses der genannten zweiten Lipaseenzyms; und
   (c) Auswählen des genannten zweiten Lipaseenzyms, worin das genannte Verhältnis im Vergleich zum ersten Lipaseenzym verändert ist.

10. Verfahren zum Ändern der nukleophilen Spezifität eines ersten Lipaseenzyms mit einer katalytischen Triade, welches Verfahren die folgenden Schritte umfaßt:
    (a) Ersetzen einer Aminosäure im ersten Lipaseenzym, die um 1-6 Aminosäuren von einer katalytischen Aminosäure der katalytischen Triade entfernt ist, durch eine davon verschiedene Aminosäure, um ein zweites Lipaseenzym herzustellen;
    (b) Bestimmen der nukleophilen Spezifität des genannten zweiten Enzyms; und
    (c) Auswählen des genannten zweiten Lipaseenzyms, worin die genannte nukleophile Spezifität im Vergleich zum ersten Lipaseenzym verändert ist.

11. Verfahren nach Anspruch 9, worin die im ersten Lipaseenzym ersetzte Aminosäure um eine Aminosäure von einer katalytischen Aminosäure der katalytischen Triade des genannten ersten Lipaseenzyms entfernt ist.

12. Verfahren nach Anspruch 10, worin die im ersten Lipaseenzym ersetzte Aminosäure um eine Aminosäure von einer katalytischen Aminosäure der katalytischen Triade des genannten ersten Lipaseenzyms entfernt ist.

17

EP 0 260 105 B1

**13.** Verfahren, das im Anschluß an die Veränderung eines Enzyms gemäß dem Verfahrens nach einem der Ansprüche 1 bis 12 und wahlweise im Anschluß an die Herstellung eines solchen Enzyms das Katalysieren einer Reaktion mit dem genannten Enzym umfaßt.

**14.** Verwendung eines Enzyms, das durch das Verfahren nach einem der Ansprüche 1 bis 12 verändert ist, in einer katalytischen Reaktion.

**15.** Herstellung des genannten Enzyms im Anschluß an die Veränderung eines Enzyms gemäß dem Verfahren nach einem der Ansprüche 1 bis 12.

**Revendications**
**Revendications pour les Etats contractants suivants : DE, FR, GB, NL, SE**

**1.** Méthode de modification du rapport de transestérification/hydrolyse ou de la spécificité nucléophile d'une enzyme ayant une triade catalytique comprenant :
a) la sélection d'une enzyme avec une triade catalytique ;
b) l'identification de la triade catalytique dans l'enzyme ;
c) la sélection d'un résidu d'acide aminé dans les 15 angströms de la triade catalytique identifiée et
d) le remplacement de l'acide aminé sélectionné par un acide aminé différent de celui présent à l'origine dans l'enzyme sélectionnée.

**2.** Méthode selon la revendication 1, qui comprend de plus l'étape de e) examiner l'enzyme ayant un acide aminé remplacé pour le rapport de transestérification/hydrolyse ou la spécificité nucléophile.

**3.** Méthode selon la revendication 2, qui comprend de plus l'étape de f) répéter c, d, et e jusqu'à ce que l'on obtienne le rapport de transestérification/hydrolyse ou la spécificité nucléophile les meilleurs souhaités.

**4.** Méthode selon l'une quelconque des revendications 1, 2 et 3, où l'enzyme est une sérine ou une cystine hydrolase.

**5.** Méthode selon l'une quelconque des revendications 1, 2 et 3, où l'enzyme est une estérase choisie parmi la protéase, une lipase ou la phospholipase.

**6.** Méthode selon la revendication 5, où l'estérase sélectionnée est une lipase.

**7.** Méthode selon la revendication 6, où l'estérase est une subtilisine.

**8.** Méthode selon la revendication 7, où la subtilisine est la subtilisine BPN'.

**9.** Méthode de modification du rapport de transestérification/hydrolyse d'une première enzyme lipase ayant une triade catalytique, une telle méthode comprenant:
(a) le remplacement d'un acide aminé dans la première enzyme lipase qui est à 1-6 acides aminés d'un acide aminé catalytique de la triade catalytique, par un acide aminé différent, pour produire une seconde enzyme lipase ;
(b) la détermination du rapport de transestérification/hydrolyse de ladite seconde enzyme lipase ; et
(c) la sélection de ladite seconde enzyme lipase où ledit rapport est modifié lorsqu'on le compare à la première enzyme lipase.

**10.** Méthode de modification de la spécificité nucléophile d'une première enzyme lipase ayant une triade catalytique, ladite méthode comprenant :
(a) le remplacement d'un acide aminé de la première enzyme lipase qui est à 1-6 acides aminés d'un acide aminé catalytique de la triade catalytique par un acide aminé différent, pour donner une seconde enzyme lipase ;
(b) la détermination de la spécificité nucléophile de ladite seconde enzyme lipase ; et
(c) la sélection de ladite seconde enzyme lipase où ladite spécificité nucléophile est modifiée lorsqu'on la compare à la première enzyme lipase.

18

**11.** Méthode selon la revendication 9, où l'acide aminé remplacé dans la première enzyme lipase est à un acide aminé d'un acide aminé catalytique de la triade catalytique de ladite première enzyme lipase.

**12.** Méthode selon la revendication 10, où l'acide aminé remplacé dans ladite première enzyme lipase est à un acide aminé d'un acide aminé catalytique de la triade catalytique de ladite première enzyme lipase.

**13.** Enzyme lipase modifiée enzymatiquement active dérivée d'une enzyme lipase précurseur ayant une triade catalytique, ladite lipase modifiée comprenant un remplacement d'un acide aminé à 1-6 acides aminés d'un acide aminé d'une triade catalytique par un acide aminé différent de l'enzyme lipase précurseur,où ladite lipase modifiée présente un changement du rapport de transestérification/hydrolyse en comparaison avec ladite enzyme lipase précurseur.

**14.** Enzyme lipase modifiée enzymatiquement active dérivée d'une enzyme lipase précurseur ayant une triade catalytique, ladite enzyme lipase modifiée comprenant un remplacement d'un acide aminé à 1-6 acides aminés d'un acide aminé de la triade catalytique par un acide aminé différent de l'enzyme lipase précurseur,où ladite enzyme lipase modifiée présente un changement de la spécificité nucléophile en comparaison avec l'enzyme lipase précurseur.

**15.** Enzyme modifiée selon la revendication 13, où l'acide aminé remplacé dans ladite enzyme lipase précurseur est à un acide aminé d'un acide aminé catalytique de la triade catalytique de ladite première enzyme lipase.

**16.** Enzyme modifiée selon la revendication 14, où l'acide aminé remplacé dans ladite enzyme lipase précurseur est à un acide aminé d'un acide aminé catalytique de la triade catalytique de ladite enzyme lipase précurseur.

**17.** Méthode qui comprend, à la suite de la modification d'une enzyme selon la méthode de l'une quelconque des revendications 1 à 12, et éventuellement la fabrication d'une telle enzyme, la catalyse d'une réaction avec ladite enzyme.

**18.** Utilisation,dans une réaction catalytique,d'une enzyme modifiée selon l'une quelconque des revendications 13 à 16.

**19.** A la suite de la modification d'une enzyme selon la méthode de l'une quelconque des revendications 1 à 12, la fabrication de ladite enzyme.

**Revendications pour l'Etat contractant suivant : ES**

**1.** Méthode de modification du rapport de transestérification/hydrolyse ou de la spécificité nucléophile d'une enzyme ayant une triade catalytique comprenant :
   a) la sélection d'une enzyme avec une triade catalytique ;
   b) l'identification de la triade catalytique dans l'enzyme ;
   c) la sélection d'un résidu d'acide aminé dans les 15 angströms de la triade catalytique identifiée et
   d) le remplacement de l'acide aminé sélectionné par un acide aminé différent de celui présent à l'origine dans l'enzyme sélectionnée.

**2.** Méthode selon la revendication 1, qui comprend de plus l'étape de e) examiner l'enzyme ayant un acide aminé remplacé pour le rapport de transestérification/hydrolyse ou la spécificité nucléophile.

**3.** Méthode selon la revendication 2, qui comprend de plus l'étape de f) répéter c, d et e jusqu'à ce que l'on obtienne le rapport de transestérification/hydrolyse ou la spécificité nucléophile les meilleurs souhaités.

**4.** Méthode selon l'une quelconque des revendications 1, 2 et 3, où l'enzyme est une sérine ou une cystine hydrolase.

**5.** Méthode selon l'une quelconque des revendications 1, 2 et 3, où l'enzyme est une estérase choisie parmi la protéase, une lipase ou la phospholipase.

**6.** Méthode selon la revendication 5, où l'estérase sélectionnée est une lipase.

**7.** Méthode selon la revendication 6, où l'estérase est une subtilisine.

**8.** Méthode selon la revendication 7, où la subtilisine est la subtilisine BPN'.

**9.** Méthode de modification du rapport de transestérification/hydrolyse d'une première enzyme lipase ayant une triade catalytique, une telle méthode comprenant :

(a) le remplacement d'un acide aminé dans la première enzyme lipase qui est à 1-6 acides aminés d'un acide aminé catalytique de la triade catalytique, par un acide aminé différent, pour produire une seconde enzyme lipase ;

(b) la détermination du rapport de transestérification/hydrolyse de ladite seconde enzyme lipase ; et

(c) la sélection de ladite seconde enzyme lipase où ledit rapport est modifié lorsqu'on le compare à la première enzyme lipase.

**10.** Méthode de modification de la spécificité nucléophile d'une première enzyme lipase ayant une triade catalytique, ladite méthode comprenant :

(a) le remplacement d'un acide aminé de la première enzyme lipase qui est à 1-6 acides aminés d'un acide aminé catalytique de la triade catalytique par un acide aminé différent, pour donner une seconde enzyme lipase ;

(b) la détermination de la spécificité nucléophile de ladite seconde enzyme lipase ; et

(c) la sélection de ladite seconde enzyme lipase où ladite spécificité nucléophile est modifiée lorsqu'on la compare à la première enzyme lipase.

**11.** Méthode selon la revendication 9, où l'acide aminé remplacé dans la première enzyme lipase est à un acide aminé d'un acide aminé catalytique de la triade catalytique de ladite première enzyme lipase.

**12.** Méthode selon la revendication 10, où l'acide aminé remplacé dans ladite première enzyme lipase est à un acide aminé d'un acide aminé catalytique de la triade catalytique de ladite première enzyme lipase.

**13.** Méthode qui comprend, à la suite de la modification d'une enzyme selon la méthode de l'une quelconque des revendications 1 à 12, et éventuellement la fabrication d'une telle enzyme, la catalyse d'une réaction avec ladite enzyme.

**14.** Utilisation dans une réaction catalytique, d'une enzyme modifiée selon l'une quelconque des revendications 1 à 12.

**15.** A la suite de la modification d'une enzyme selon la méthode de l'une quelconque des revendications 1 à 12, la fabrication de ladite enzyme.